(19) Europäisches Patentamt
European Patent Office
Office européen des brevets

(11) Veröffentlichungsnummer: **0 301 447 B1**

(12) # EUROPÄISCHE PATENTSCHRIFT

(45) Veröffentlichungstag der Patentschrift: **15.04.92**

(51) Int. Cl.⁵: **C08F 120/60**, C08F 120/36, C02F 1/54, C08F 126/02, H01B 1/12, A61K 7/06

(21) Anmeldenummer: **88111908.5**

(22) Anmeldetag: **23.07.88**

(54) **Polymere aus alkoxylierten ungesättigten quartären Ammoniumsalzen, ihre Herstellung und Verwendung.**

(30) Priorität: **31.07.87 DE 3725449**
**31.07.87 DE 3725427**

(43) Veröffentlichungstag der Anmeldung:
**01.02.89 Patentblatt 89/05**

(45) Bekanntmachung des Hinweises auf die Patenterteilung:
**15.04.92 Patentblatt 92/16**

(84) Benannte Vertragsstaaten:
**DE ES FR GB IT NL**

(56) Entgegenhaltungen:
**DE-A- 2 430 286**
**GB-A- 1 139 099**

(73) Patentinhaber: **BASF Aktiengesellschaft**
**Carl-Bosch-Strasse 38**
**W-6700 Ludwigshafen(DE)**

(72) Erfinder: **Goertz, Hans-Helmut, Dr.**
**Am Wurmberg 11**
**W-6713 Freinsheim(DE)**
Erfinder: **Oftring, Alfred, Dr.**
**Berner Weg 26**
**W-6700 Ludwigshafen(DE)**
Erfinder: **Vogel, Friedrich, Dr.**
**Am Boehlig 13**
**W-6706 Wachenheim(DE)**

EP 0 301 447 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Polymere, die durch Polymerisation von wasserlöslichen Monomeren, die neben mindestens einer polymerisierbaren olefinischen Doppelbindung ein quartäres Stickstoffatom enthalten, das mit mindestens einer Hydroxyalkylgruppe substituiert ist und die als halogenidfreie Salze vorliegen, und ggf. mit weiteren Comonomeren erhalten werden, ihre Herstellung und Verwendung.

In der DE-A-24 30 286 werden Ammoniumchlorhydringruppen enthaltende Polymerisate beschrieben, welche Einheiten der allgemeinen Formel

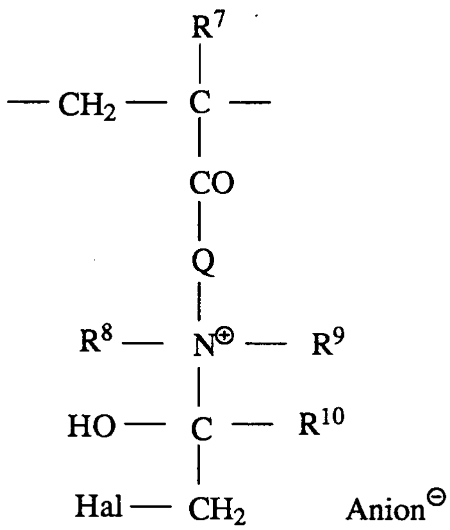

in der $R^7$ Wasserstoff oder Methyl, $R^8$ und $R^9$ Alkyl, Cycloalkyl oder Aryl, $R^{10}$ Wasserstoff, Methyl oder Phenyl, Hal Chlor oder Brom und Q eine Oxyalkylen-oder Aminoalkylen-Gruppierung bedeuten, enthalten. Diese Polymerisate können für die Herstellung von Formkörpern, Überzügen oder Klebemitteln, zum Veredeln von Textilien oder als Lacke verwendet werden.

In der EP-A2-00 98 802 wird die Umsetzung von Trialkylaminen mit Ethylenoxid in Gegenwart von Säuren im wäßrigen Medium zu quartären Ammoniumsalzen beschrieben. Eine Ouaternierung von tertiären Aminen, die eine polymerisierbare olefinische Doppelbindung enthalten, mit Ethylenoxid oder einem anderen Alkylenoxid geht daraus nicht hervor.

G.G. Skvortsova et al. (Khim Geterots. Soed. 777-780 (1973), engl. Übersetzung Seiten 713-716) beschreiben die Umsetzung von N-Vinylazolen, z.B. N-Vinylimidazol, mit Halogenhydrinen zu entsprechenden Vinylimidazoliumsalzen. Diese Methode erlaubt jedoch nur die Gewinnung von Salzen, die Halogenid als Gegenion enthalten, oder es müßte ein aufwendiger Anionenaustausch vorgenommen werden. Gemäß dem Verfahren dieser Literaturstelle wird das N-Vinylimidazol im verschlossenen Gefäß direkt mit überschüssigem Ethylenchlorhydrin durch 22-stündiges Erhitzen bei 90 ° C umgesetzt. Bereits bei Temperaturen um 70 ° C und Reaktionszeiten von 50 Stunden findet praktisch keine Umsetzung zum quaternären Salz mehr statt. Auch mit substituierten Chlorhydrinen können keine vernünftigen Ausbeuten erhalten werden, und mit Fluorhydrin findet selbst durch 100-stündiges Erhitzen bei 90 ° C keine Umsetzung statt.

Die Einschränkung, daß für technische Polymerisations-Verfahren nur Salze, die ein Halogenid als Anion enthalten. beschrieben sind, ist für viele Fälle mißlich. Das Halogenid kann Ursache für schwerwiegende Korrosionsprobleme sein, beispielsweise bei Verfahren zur Herstellung kationischer Polymerer in deren Gegenwart. Es ist daher wünschenswert, auf halogenidfreie Monomere zur Herstellung halogenidfreier kationischer Polymerisate für großtechnische Verfahren zurückgreifen zu können. Dabei ist es vorteilhaft, wenn das Gegenion keine korrosive sondern sogar eine schützende Wirkung aufweist, wie es beispielsweise von Phosphit, Phosphat u.a. bekannt ist.

Aufgabe der Erfindung ist es, wasserlösliche kationische Polymere mit beliebigen Anionen außer den korrosiven Halogeniden, die aus halogenidfreien quartären Monomeren mit mindestens einer polymerisierbaren olefinischen Doppelbindung und einem quartären Stickstoffatom, das mit mindestens einer Hydroxyalkylgruppe substituiert ist, und ggf. weiteren Comonomeren hergestellt werden, zur Verfügung zu stellen und die insbesondere als Leitfähigkeitsharze als Flockungsmittel oder in kosmetischen Zubereitungen Verwendung finden sollen.

Hierzu werden wasserlösliche Monomere mit einer polymerisierbaren olefinischen Doppelbindung und einem quartären Stickstoffatom der Formel I

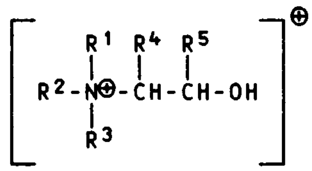 $A^{\ominus}$ I,

in der

$R^1$ einen Alkenylrest mit 2 bis 4 C-Atomen, bevorzugt Allyl oder Methallyl, oder einen Rest der Formel II

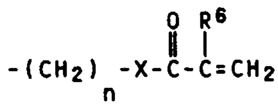 II,

in dem für n eine ganze Zahl von 1 bis 18, bevorzugt 2 bis 6, für X ein Sauerstoffatom oder eine NH-Gruppe und für $R^6$ ein Wasserstoffatom oder Methyl stehen,

$R^2$ einen Alkylrest mit 1 bis 18 C-Atomen, bevorzugt 1 bis 4 C-Atomen, oder einen Hydroxyalkylrest mit 2 bis 18 C-Atomen, bevorzugt 2 bis 4 C-Atomen,

$R^3$ einen Alkenylrest mit 2 bis 4 C-Atomen, bevorzugt Allyl oder Methallyl, oder einen Alkylrest mit 1 bis 18 C-Atomen, bevorzugt 1 bis 4 C-Atomen, oder einen Hydroxyalkylrest mit 2 bis 18 C-Atomen, bevorzugt 2 bis 4 C-Atomen, bedeuten

oder die Reste $R^1$, $R^2$ und $R^3$ zusammen mit dem quartären Stickstoffatom einen 5- oder 6-gliedrigen heterocyclischen Ring bilden, der durch eine polymerisationsfähige olefinische Doppelbindung, bevorzugt eine Vinylgruppe, substituiert ist, ggf. ein weiteres Stickstoffatom und ggf. ein oder zwei Alkylreste mit 1 bis 4 C-Atomen als weitere Substituenten enthält

und $R^4$ und $R^5$ ein Wasserstoffatom oder einen Alkylrest mit 1 bis 28 C-Atomen, bevorzugt 1 bis 16 C-Atomen, oder eine durch einen Alkoxyrest mit 1 bis 10 C-Atomen im Alkyl substituierte Methylgruppe bedeuten

und $A^{\ominus}$ für das Äquivalent eines Anions außer Halogenid steht, als Ausgangsmonomere verwendet.

Die Herstellung der halogenidfreien Monomeren ist nicht Gegenstand der Erfindung. Sie wird aber im folgenden näher erläutert, da ein großtechnisches Verfahren für ihre Herstellung nicht beschrieben ist.

Im einzelnen wird zu der Formel I ausgeführt:

Alkenylreste mit 2 bis 4 C-Atomen für $R^1$ und $R^3$ stellen insbesondere Vinyl, Allyl oder Methallyl dar.

Bei dem Rest der Formel II für $R^1$ handelt es sich um Reste, die sich von Acrylsäure- und Methacrylsäureestern oder -amiden ableiten. Dabei sind für n die Zahlen 2 und 3 besonders bevorzugt.

Die Alkylreste mit 1 bis 18 C-Atomen für $R^2$ und $R^3$, sind geradkettig oder verzweigt und es können beispielsweise genannt werden Methyl, Ethyl, Propyl, Isopropyl, Butyl, Isobutyl, Octyl, Lauryl, Cetyl und Stearyl.

Von den hervorzuhebenden Alkylresten mit 1 bis 4 C-Atomen sind insbesondere Methyl und Ethyl zu nennen.

Als Hydroxyalkylreste für $R^2$ und $R^3$, geradkettig oder verzweigt, können beispielsweise Hydroxyethyl, Hydroxypropyl, Hydroxybutyl, Hyroxyoctyl, Hydroxydodecyl, Hydroxyhexadecyl und Hydroxyoctadecyl, genannt werden.

Von den hervorzuhebenden Hydroxyalkylresten mit 2 bis 4 C-Atomen sind insbesondere Hydroxyethyl und Hydroxypropyl, wie sie bei der Herstellung als Umsetzungsprodukte von Ethylenoxid und Propylenoxid entstehen, zu nennen.

Für den Fall, daß die Reste $R^1$, $R^2$ und $R^3$ zusammen mit dem quartären Stickstoffatom einen durch eine Vinylgruppe substituierten heterocyclischen Ring bilden, enthält die Verbindung der Formel I, insbesondere ein 2- oder 4-Vinylpyridin oder einen N-Vinylimidazolring, der ggf. an den C-Atomen durch ein oder zwei Alkylreste mit 1 bis 4 C-Atomen, bevorzugt Methyl, substituiert ist, eingebaut.

Bevorzugt handelt es sich dabei um das N-Vinylimidazol und das N-Vinyl-2-methyl-imidazol.

Die Substituenten $R^4$ und $R^5$ bedeuten insbesondere Wasserstoffatome, wobei bevorzugt nur einer der

Reste $R^4$ oder $R^5$ durch einen Alkylrest oder einen Alkoxymethylrest ersetzt ist.

Als Alkylreste für $R^4$ und $R^5$ sind beispielsweise Methyl, Ethyl, Hexyl, Decyl, Tetradecyl und Hexadecyl zu nennen. Hervorzuhebende Reste sind Methyl, Ethyl oder Tetradecyl.

Als Alkoxymethylreste sind beispielsweise Methoxymethyl, Ethoxymethyl, Propoxymethyl, Butoxymethyl, Isopropoxymethyl, Isobutoxymethyl, 2-Ethylhexoxymethyl, Octoxymethyl und Decyloxymethyl zu nennen und Methoxymethyl, Ethoxymethyl oder 2-Ethylhexoxymethyl hervorzuheben.

Das Anion ist das Äquivalent eines Anions außer Halogenid. Insbesondere kommen in Betracht Phosphat, Sulfat, Nitrat, Phosphit, Hypophosphit und Phosphonat. Bei mehrbasigen anorganischen oder organischen Säuren kommen selbstverständlich auch die Anionen der einzelnen Dissoziationsstufen in Betracht, wie Phosphat, Hydrogenphosphat und Dihydrogenphosphat. Als Äquivalente einer organischen Säure kommen insbesondere in Betracht Anionen von einbasigen Alkancarbonsäuren mit 1 bis 18 C-Atomen, ggf. im Alkylrest durch eine Hydroxygruppe substituiert, von zwei- oder dreibasigen gesättigten organischen Carbonsäuren mit insgesamt 2 bis 10 C-Atomen und ggf. durch eine oder mehrere Hydroxigruppen substituiert, und von aromatischen Carbonsäuren, wie beispielsweise Formiat, Acetat, Oxalat, Benzoat, Lactat, Glykolat, Malat, Citrat und Tartrat.

Aus den hervorgehobenen und bevorzugten Bedeutungen ergibt sich, daß die bevorzugten Verbindungen der Formel I sich ableiten von basischen Estern und von durch basische Reste substituierten Amiden der Acryl- und Methacrylsäure, von Allylaminderivaten und von Vinylimidazolverbindungen.

Die Verbindungen der Formel I werden hergestellt, indem man ein Amin der Formel III

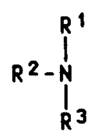

III,

in der $R^1$, $R^2$ und $R^3$ die für Formel I genannten Bedeutungen aufweisen, mit einem Epoxid det Formel IV

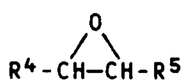

IV,

in der $R^4$ und $R^5$ die für Formel I genannten Bedeutungen aufweisen,
in wäßriger Lösung in Gegenwart einer dem Anion A⊖ entsprechenden Säure in einem pH-Bereich von 7 bis 10 bei Temperaturen von 10 bis 90 °C umsetzt.

Die Amine der Formel III sind bekannt oder können nach üblichen Methoden hergestellt werden. Es handelt sich um tertiäre Amine, die mit einem Epoxid der Formel IV umgesetzt werden.

In Ausnahmefällen kann von einem sekundären oder primären Amin ausgegangen werden, und zwar wenn einer der Reste $R^2$ oder $R^3$ oder beide einen Hydroxyalkylrest darstellen, der identisch ist mit dem die Substituenten $R^4$ und $R^5$ tragenden Hydroxyethylrest.

Für die Umsetzung eines Amins der Formel III mit einem Epoxid der Formel IV liegt der bevorzugte pH-Bereich bei 8,5 bis 9,5 und die bevorzugte Temperaturen bei 40-70 °C, wobei 1 bis 2 Mol, vorzugsweise 1 bis 1,5 Mol Epoxid pro Mol Amin der Formel III verwendet werden.

Für den Fall, daß ein sekundäres Amin eingesetzt wird, werden 2 bis 3, vorzugsweise 2 bis 2,5 Mol Epoxid eingesetzt. Bei primären Aminen verwendet man 3 bis 4 Mol, bevorzugt 3 bis 3,5 Mol Epoxid.

Die Umsetzung wird zweckmäßigerweise im wäßrigen Medium unter Normaldruck oder ggf. erhöhtem Druck durchgeführt, wobei die Konzentration der Reaktionsteilnehmer, bezogen auf das Gesamtgewicht der Reaktionsmischung, zweckmäßig bei 10 bis 80, insbesondere 20 bis 70 Gew.% liegt.

Die Reaktion kann in der Weise durchgeführt werden, daß man das Amin und das Epoxid in Wasser zusammen vorlegt und die Reaktion bis zur pH-Wert-Konstanz bei einem mit der jeweiligen Säure festzuhaltenden pH-Wert durchführt, oder aber, und dies vorzugsweise, das Epoxid in reiner Form oder in Wasser gelöst bzw. emulgiert bei einem bestimmten pH-Wert zu einer wäßrigen Aminlösung zutropft.

Gegebenenfalls wird nach Beendigung der Umsetzung durch Zugabe von dem Anion A⊖ entsprechender Säure aus der Ammoniumbase das Ammoniumsalz erhalten.

Die erhaltenen quartären ungesättigten Ammoniumverbindungen können vorzugsweise direkt in wäßriger Lösung weiter verwendet oder aber beispielsweise durch Sprühtrocknung oder Ausfrieren isoliert werden.

Aufgrund der obengenannten bevorzugten Bedeutungen sind die bevorzugt umzusetzenden Amine der Formel III die Verbindungen der Formel V

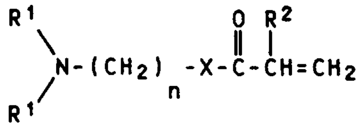

V,

in der $R^1$ einen Alkylrest mit 1 bis 4 C-Atomen, $R^2$ ein Wasserstoffatom oder einen Methylrest, X ein Sauerstoffatom oder eine NH-Gruppe und n 2 oder 3 bedeuten, oder Allylamine der Formel VI

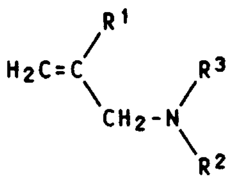

VI,

in der $R^1$ ein Wasserstoffatom oder eine Methylgruppe, $R^2$ einen Alkylrest mit 1 bis 4 C-Atomen oder einen Hydroxyalkylrest mit 2 bis 4 C-Atomen und $R^3$ einen Allyl- oder Methallylrest oder einen Alkylrest mit 1 bis 4 C-Atomen oder einen Hydroxyalkylrest mit 2 bis 4 C-Atomen bedeuten, oder
Vinylimidazole der Formel VII

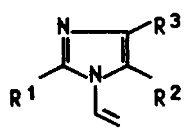

VII,

in der $R^1$, $R^2$ und $R^3$ ein Wasserstoffatom, wobei für einen der Reste $R^1$ bis $R^3$ Methyl stehen kann, bedeuten.

Bei den bevorzugten Epoxiden der Formel IV bedeuten $R^4$ und $R^5$ Wasserstoff oder einer der Rest $R^4$ oder $R^5$ Methyl, Ethyl oder Tetradecyl sowie Methoxymethyl, Ethoxymethyl oder 2-Ethylhexoxymethyl.

Dementsprechend sind als Ausgangsverbindungen für Monomere der Formel I beispielsweise zu nennen Dimethylaminoethylacrylat, Dimethylaminoethylmethacrylat, Dietylaminoethylacrylat, Diethylaminoethylmethacrylat und Dimethylaminopropylacrylamid, N-Methyldiallylamin, Allylamin und Diallylamin, N-Vinylimidazol sowie N-Vinyl-2-methylimidazol.

Als Epoxide sind beispielsweise zu nennen Ethylenoxid, Propylenoxid, Butylenoxid-1 und -2, (2-Ethylhexyl)-(propylen-2-oxiran)-ether, 1,2-Epoxyhexadecan, Methoxymethyloxiran und Ethoxymethyloxiran.

Bevorzugte Anionen sind beispielsweise Phosphat, Sulfat sowie Formiat, Acetat, Glykolat, Oxalat, Lactat und Citronat, ggf. in Form von Hydrogenanionen.

Beispiele für die Herstellung Monomerer der Formel I

Beispiel 1

Zu einer Lösung von 94 g (1 Mol) N-Vinylimidazol in 150 g Wasser tropft man innerhalb von 3,5 Stunden bei 40 °C 45 g (1,02 mol) Ethylenoxid zu. Der pH-Wert wird mittels 85%iger Phosphorsäure zwischen 9 und 9,5 gehalten.

Anschließend rührt man noch 4 Stunden bei 40 °C und bei einem pH-Wert von 9 weiter. Nach dieser Zeit konnte chromatographisch kein Vinylimidazol sowie potentiometisch kein Ethylenoxid mehr festgestellt werden.

Mit Phosphorsäure wird anschließend ein pH-Wert von 5,5 eingestellt. Es werden insgesamt 60 g 85%ige Phosphorsäure verbraucht. Man erhält 345 g einer ca. 55%igen klaren wäßrigen Lösung des Ammoniumsalzes der Formel

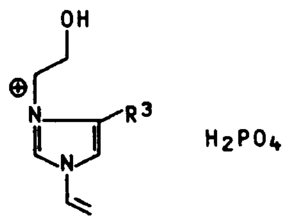

Anstelle von Phosphorsäure können analog andere in der Beschreibung genannte Säuren verwendet werden, wie Schwefelsäure, Milchsäure und Zitronensäure.

Beispiel 2

Beispiel 1 wird analog mit 81 g (1,4 mol) Propylenoxid anstelle vom Ethylenoxid in 700 g Wasser durchgeführt.

Nach dem Reaktionsende wird die klare Lösung von nicht umgesetztem Oxid mittels Vakuumdestillation befreit.

Man erhält 420 g einer ca. 57 %igen Lösung des Ammoniumsalzes der Formel

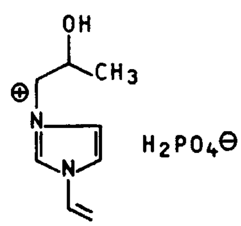

Beispiel 3

Zu einer Lösung von 157,2 g (1 mol) Dimethylaminoethylmethacrylat in 700 g Wasser werden bei 45 ° C 62 g (1,4 mol) Ethylenoxid innerhalb von 1,5 Stunden zugetropft, wobei der pH-Wert mit 85 %iger Phosphorsäure bei 9 - 9,5 gehalten wird. Anschließend wird 2 Stunden bei 45 ° C und einem pH von 9 nachgerührt. Die resultierende Lösung wird mit Phosphorsäure auf einen pH-Wert von 5 eingestellt. Man erhält 986 g einer ca. 25%igen wäßrigen Lösung des Ammoniumsalzes der Formel

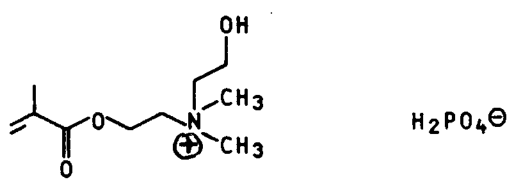

Beispiel 4

Zu einer Emulsion von 83,1 g (1 mol) Methyldiallylamin in 700 g Wasser tropft man bei 35 ° C 62 g (1,4 mol) Ethylenoxid in 4 Stunden zu. Der pH-Wert wird mit 25%iger Schwefelsäure bei 9,5 gehalten. Nach 5-stündigem Weiterrühren bei 40 ° C und einem pH-Wert von 9,0 wird mit Schwefelsäure ein pH-Wert von 5 eingestellt.

Nach dem Aufkonzentrieren der wäßrigen klaren Lösung resultierten 330 g einer ca. 55%igen Lösung der Verbindung

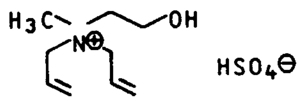

Beispiel 5

Man legt 47 g (0,5 mol) N-Vinylimidazol und 93 g (0,5 mol) (2-Ethylhexyl)-(propylen-2-oxiran)-ether in 400 g Wasser vor.

Mit 85%iger Phosphorsäure wird ein pH-Wert von 8,5 während 5 Stunden bei 60 °C gehalten. Nach dem Einstellen des pH auf 5 resultieren 565 g einer ca. 30%igen klaren wäßrigen Lösung von

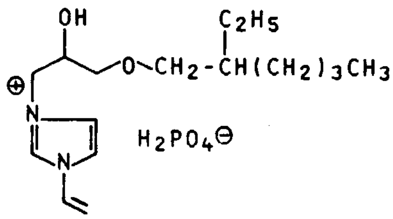

Die Monomeren der Formel I lassen sich vorteilhaft zur Herstellung von wasserlöslichen kationischen Polymeren in Form von Homopolymerisaten oder Copolymerisaten verwenden.

Gegenstand der Erfindung sind daher Polymere, die durch Polymerisation von Monomeren der Formel I erhalten werden als wasserlösliche Homopolymerisate oder als wasserlösliche Copolymerisate aus 2 bis 98 Gew.-% eines Monomeren der Formel I und 98 bis 2 Gew.% eines polymerisierbaren wasserlöslichen ungesättigten Comonomeren, bezogen auf das Gesamtgewicht der Monomeren, ihre Herstellung durch radikalische Polymerisation, insbesondere in wäßriger Lösung, und ihre Verwendung.

Entsprechend den Ausführungen über die Monomeren der Formel I sind Homo- und Copolymerisate mit den hervorgehobenen und bevorzugten Bedeutungen für die Formel I bevorzugt.

Als Homopolymerisate können beispielsweise genannt werden

Poly-[2-(dimethyl-hydroxyethyl-ammonio)-ethylmethacrylat-dihydrogenphosphat] und Poly-(N-Vinyl-N′-hydroxypropyl-imidazolium-dihydrogenphosphat) sowie
Poly-[2-(Hydroxyethyl-dimethyl-ammonio)-ethylmethacrylat-sulfat] und das entsprechende -phosphat,
Poly-[2-(hydroxypropyl-dimethyl-ammonio)-ethylmethacrylat-hydrogenphosphat],
Poly-[2-(hydroxyethyl-diethyl-ammonio)-ethyl-acrylat-lactat] und das entsprechende -phosphat,
Poly-[2-(hydroxypropyl-diethyl-ammonio)-ethylacrylat-acetat] und das entsprechende -phosphat,
Poly-[2-hydroxyethyl-dimethyl-ammonio)-propylmethacrylat-phosphat],
Poly-[N-(hydroxybutyl-dimethyl-ammonio)-propylmethacrylamid-citrat] und das entsprechende -phosphat,
Poly-[diallyl-dihydroxyethyl-ammonium-phosphit] und das entsprechende -phosphat,
Poly-[diallyl-methyl-hydroxyethyl-ammonium-phosphat],
Poly-[allyl-tris(hydroxypropyl-ammonium-succinat] und das entsprechende -phosphat,
Poly-[1-vinyl-3-hydroxybutyl]-imidazolium-phosphat],
Poly-[1-vinyl-3-hydroxyethyl-imidazolium-phosphat] und
Poly-[1-vinyl-2-methyl-3-hydroxypropyl-imidazolium-benzoat und das entsprechende phosphat].

Bevorzugt handelt es sich um Poly-(diallyl-hydroxyethyl-methyl-ammonium-hydrogensulfat) und Poly-(N-Vinyl-N′-hydroxyethyl-imidazolium-dihydrogenphosphat).

Die bei der Herstellung von Copolymeren als wasserlösliche, ungesättigte Comonomere verwendeten Monomeren sind beispielsweise die den quartären Monomeren der Formel I zugrundeliegenden tertiären Amine der Formel III, wie Dimethylaminoethylacrylat, Dimethylaminoethylmethacrylat, Dietylaminoethylacrylaat, Diethylaminoethylmethacrylat und Dimethylaminopropylacrylamid, N-Methyldiallylamin, Allylamin und Diallylamin, N-Vinylimidazol sowie N-Vinyl-2-methylimidazol.

Weiterhin seien beispielsweise genannt Acrylamid und Methacrylamid, N-Methylolacrylamid und N-Methylolmethacrylamid, Hydroxyalkylacryalate und Hydroxyalkylmethacrylate mit 2 bis 4 C-Atomen im Hydroxyalkylrest, wie Hydroxyethyl(meth)acrylat, Hydroxypropyl(meth)acrylat, gegebenenfalls in Form ihrer technischen Gemische, und Polyethylenglykol(meth)acrylate mit 2 bis 50 Ethylenoxideinheiten.

Als bevorzugte Comonomere kommen N-Vinylamide, wie N-Vinylpyrrolidon, N-Vinylcaprolactam, N-Vinylformamid, N-Vinylacetamid und N-Methyl-N-vinylacetamid und Hydroxyalkylacrylate und -methacrylate mit 2 bis 4 C-Atomen im Hydroxyalkylrest in Betracht.

Gegebenenfalls können auch wasserunlösliche Comonomere, wie Styrol, α-Olefine, wie Penten oder Buten, Acryl- und Methacrylsäurealkylester mit 1 bis 18 C-Atomen im Alkylrest, Carbonsäurevinylester mit 2 bis 10 C-Atomen im Carbonsäurerest, wie Vinylacetat oder Vinylpropionat, mit den Monomeren der Formel I copolymerisiert werden.

Diese Comonomeren können in einer Menge von 5 bis 50 Gew.%, bezogen auf das Gesamtgewicht der Monomeren, einpolymerisiert werden.

Besonders bevorzugt ist die Verwendung der Monomeren der Formel I zur Herstellung von Homopolymerisaten oder von wasserlöslichen Copolymerisaten aus 98 bis 2 Gew.% eines Monomeren der Formel I mit 2 bis 98 Gew.% eines Monomeren aus der Gruppe N-Vinylpyrrolidon, 2-Hydroxyethylacrylat, 2-Hydroxypropylacrylat (technisches Gemisch), bezogen auf das Gesamtgewicht der Monomeren.

Die Herstellung der Homo- und Copolymerisate erfolgt durch an sich übliche radikalische Polymerisation insbesondere in wäßriger Lösung.

Die Polymerisationen werden zweckmäßigerweise in 10 bis 70 gew.%iger wäßriger Lösung in Gegenwart von radikalbildenden Initiatoren in einer Menge von 0,1 bis 3,0 %, bezogen auf das Gewicht der Monomeren, und bei Temperaturen von 40 bis 100° C durchgeführt.

Als radikalbildende Initiatoren können Wasserstoffperoxid oder anorganische Persulfate verwendet werden, ebenso organische Verbindungen vom Peroxid- oder Azotyp. Als organische Peroxide kommen beispielsweise Dicyclohexylperoxidicarbonat, Dibenzoylperoxid, Dilauroylperoxid, tert.-Butylperpivalat, tert.-Butyl-2ethylhexanoat und als Azoverbindungen 2,2′-Azobis(2-amidinopropan)hydrochlorid oder 2,2′-Azobis-(4-cyanopentansäure) in Betracht.

Eine Wasserlöslichkeit der verwendeten Radikalbildner ist nicht zwingend erforderlich. Wasserunlösliche Initiatoren können beispeispielsweise in einem niederen Alkohol gelöst oder ohne Lösungsmittel direkt zudosiert werden. Die Wahl des Initiators richtet sich zweckmäßigerweise u.a. nach der Polymerisationstemperatur, die bevorzugt zwischen 40 und 100° C liegt.

Das Molekulargewicht des Polymeren kann, falls wünschenswert, z.B. durch Zugabe von Reglern in die Reaktionsmischung in üblicher Weise gesteuert werden. Als Regler kommen beispielsweise niedere Alkohole in Betracht. Es können aber auch andere hierfür übliche Verbindungen, wie Schwefelverbindungen, beispielsweise 2-Mercaptoethanol, Butylmercaptan, Dodecylmercaptan, Thioglykolsäure, Thioessigsäure, Thiomilchsäure Halogenverbindungen, wie Tetrachlorkohlenstoff, 1,1,1-Tribrompropan, oder Ameisensäure und ihre Derivate eingesetzt werden.

Durch die geeignete Wahl von Regler, Initiator, Polymerisationstemperature und Monomerenkonzentration wird der K-Wert des erhaltenen Polymerisates, der ein Maß für das Molekulargewicht ist, eingestellt. Die K-Werte der erhaltenen Homo- und Copolymerisate liegen zweckmäßigerweise zwischen 10 und 150, gemessen an einer 1 gew.-%igen wäßrigen Lösung bei 25° C nach Fikentscher.

Die erhaltenen Polymere können vielfältig verwendet werden, beispielsweise als Leitfähigkeitsharze, als Flockungsmittel, in der Kosmetik z.B. als Haarkonditionierungsmittel, als Hilfsmittel bei der Ölgewinnung und anderes mehr.

Davon ist die Verwendung in der Kosmetik besonders hervorzuheben. Bei der Verwendung in Haarspraydosen kann nachgewiesen werden, daß gegenüber handelsüblichen kationischen Polymeren eine deutliche Überlegenheit im Hinblick auf Korrosion existiert. Eine praktische Formulierung für einen kosmetischen Haarschaum kann dem Verwendungsbeispiel entnommen werden. Mit dieser Formulierung wird eine gute Naß- und Trockenkämmbarkeit erreicht.

Beispiele für die Herstellung von erfindungsgemäßen Polymeren

Beispiel 6

Eine Mischung von 115 g N-Vinylpyrrolidon, 148 g Monomerlösung gemäß Beispiel 1 und 130 ml Wasser wird mit ca. 70 ml 10 gew.%iger Natronlauge auf pH 8.0 eingestellt und dient als Zulauf 1. Zulauf 2 besteht aus 1,37 g tert.-Butylperpivalat in 50 g Isopropanol. In einer 1 l Glasapparatur werden 150 ml Wasser, 30 ml Zulauf 1 und 3 ml Zulauf 2 vorgelegt und unter Rühren bei 70° C 10 min anpolymerisiert. Anschließend werden bei dieser Temperatur Zulauf 1 in 4 h und Zulauf 2 in 6 h zugegeben und eine Stunde nachgerührt. Nach dem Abdestillieren des Isopropanols erhält man eine leicht trübe Lösung eines Polymeren mit K-Wert 66, die beim weiteren Verdünnen mit Wasser klar wird.

Beispiel 7

120 g einer Monomerlösung gemäß Beispiel 3 werden mit 50 ml Wasser verdünnt und mit ca. 20 ml 10 gew.%iger Natronlauge auf pH 8,5 eingestellt. Die erhaltene Lösung dient als Zulauf 1. 120 g N-Vinylpyrrolidon dienen als Zulauf 2. Zulauf 3 ist eine Lösung von 0,75 g tert.-Butylperpivalat in 50 g Isopropanol. In einer 1 l-Glasapparatur, die mit Rührer und Zulaufgefäßen ausgestattet ist, werden 175 g Wasser, 15 ml Zulauf 1, 10 ml Zulauf 2 und 2,5 ml Zulauf 3 vorgelegt und unter Rühren bei 70 °C 10 min anpolymerisiert. Anschließend werden bei dieser Temperatur die Zuläufe 1 und 2 in 4 h, Zulauf 3 in 6 h zugegeben und 1 h nachgerührt. Nach dem Abdestillieren des Isopropanols erhält man eine trübe 35 %ige Lösung eines Polymeren mit K-Wert 61.

Beispiel 8

Eine Mischung aus 120 g Monomerlösung gemäß Beispiel 2, 30 g Hydroxypropylacrylat und 60 g Wasser wird mit ca. 20 ml 10%iger Natronlauge auf pH 7.0 eingestellt und dient als Zulauf 1. Zulauf 2 ist eine Lösung von 0,7 g tert.-Butylperpivalat in 50 g Isopropanol. In einer 0,5 l-Glasapparatur, die mit Rührer und Zulaufgefäßen ausgestattet ist, werden 60 ml Wasser, 20 ml Zulauf 1 und 3 ml Zulauf 2 vorgelegt und bei 70 °C 10 min anpolymerisiert. Anschließend werden bei dieser Temperatur Zulauf 1 in 4 und Zulauf 2 in 6 h zugegeben und 1 h nachgerührt. Nach Abdestillieren des Isopropanols erhält man eine fast klare Lösung eines Polymeren von K-Wert 28.

Beispiel 9

In 100 g einer Monomerlösung gemäß Beispiel 4 werden 1,6 g 2,2′-Azobis(2-amidinopropan)-hydrochlorid gelöst und unter Rühren 4 h auf 70 °C erwärmt. Man erhält eine Polymerlösung von K-Wert 14.

Beispiel 10

125 g einer Monomerlösung gemäß Beispiel 5 werden mit 66 g N-Vinylpyrroldion und 125 g Wasser gemischt und mit ca. 10 ml 10%iger Natronlauge auf pH 8.0 eingestellt. Die Lösung dient als Zulauf 1. Zulauf 2 ist eine Lösung von 0,7 g tert.-Butylperpivalat in 50 g Isopropanol. In einer 0,5 l Glasapparatur werden 65 g Wasser, 20 ml Zulauf 1 und 3 ml Zulauf 2 vorgelegt und bei 70 °C unter Rühren 10 min anpolymerisiert. Anschließend werden Zulauf 1 in 4 h und Zulauf 2 in 6 h bei derselben Temperatur zugegeben und noch eine Stunde nachgerührt. Nach Abdestillieren des Isopropanols erhält man eine trübe Polymerlösung.

Beispiel 11

Eine Mischung aus 123 g einer 52 %igen Monomer-Lösung, die analog Beispiel 1, jedoch unter Verwendung von Schwefelsäure statt Phosphorsäure hergestellt wurde, 90 g N-Vinylpyrrolidon, 0,3 g 2-Mercaptoethanol und 140 ml Wasser wird mit ca. 7 ml 10 %iger Natronlauge auf pH 7,5 eingestellt und dient als Zulauf 1. Zulauf 2 ist eine Lösung von 1,1 g 2,2′-Azobis(2-amidinopropan)hydrochlorid in 50 ml Wasser. In einer 1 l Glasapparatur, die mit Rühren und Zulaufgefäßen ausgestattet ist, werden 105 ml Wasser, 20 ml Zulauf 1 und 2 ml Zulauf 2 vorgelegt und unter Rühren bei 65 °C 10 min anpolymerisiert. Dann werden Zulauf 1 in 4 h und Zulauf 2 in 6 h bei der gleichen Temperatur zudosiert und anschließend noch 1 h nachgerührt. Man erhält eine leicht trübe Lösung eines Polymeren vom K-Wert 54, die beim Verdünnen auf 20 Gew.-% völlig klar wird.

Beispiel 12

Eine Mischung aus 112 g einer 70 %igen Lösung eines Monomeren, das analog Beispiel 1 hergestellt wurde, jedoch unter Verwendung von Milchsäure statt Phosphorsäure, 115 g N-Vinylpyrroldion, 0,39 g 2-Mercaptoethanol und 58 ml Wasser wird mit ca. 17 ml 10 %iger Natronlauge auf pH 7,5 eingestellt und dient als Zulauf 1. Zulauf 2 ist eine Lösung von 1,36 g 2,2′-Azobis(amidinopropan)hydrochlorid in 30 ml Wasser. In einer 1 l-Glasapparatur mit Rührer und Zulaufgefäßen werden 150 ml Wasser, 20 ml Zulauf 1 und 2 ml Zulauf 2 vorgelegt und bei 65 °C unter Rühren 10 min anpolymerisiert. Dann werden bei dieser Temperatur Zulauf 1 in 4 h und Zulauf 2 in 6 h zudosiert und noch 1 h nachgerührt. Man erhält eine Lösung

eines Polymeren vom K-Wert 59.

Beispiel 13

Eine Mischung aus 156 g einer 57 %igen Lösung eines Monomeren, das analog Beispiel 1 hergestellt wurde, jedoch unter Verwendung von Zitronensäure statt Phosphorsäure, 90 g N-Vinylpyrrolidon, 0,36 g 2-Mercaptoethanol und 35 ml Wasser wurde mit ca. 25 ml 10%iger Natronlauge auf pH 7,5 eingestellt und dient als Zulauf 1. Zulauf 2 ist eine Lösung von 1,25 g 2,2'-Azobis(2-amidinopropan)hydrochlorid in 50 ml Wasser. In einer 1 l-Glasapparatur, die mit Rührer und Zulaufgefäß ausgerüstet ist, werden 90 ml Wasser, 20 ml Zulauf 1 und 2 ml Zulauf 2 vorgelegt und unter Rühren bei 65 °C 10 min anpolymerisiert. Dann werden Zulauf 1 in 4 h und Zulauf 2 in 6 h zugegeben und noch 1 h nachgerührt. Man erhält eine leicht trübe Lösung eines Polymeren vom K-Wert 46, die beim Verdünnen auf 20 Gew.% völlig klar wird.

Beispiel 14

350 g einer Monomerlösung gemäß Beispiel 1 werden mit 150 ml 10%iger Natronlauge auf pH 8.0 eingestellt und dienen als Zulauf 1. Zulauf 2 ist eine Lösung von 1,35 g tert.-Butylperpivalat in 50 g Isopropanol. In einer 1 l-Glasapparatur werden 100 ml Wasser, 90 ml Zulauf 1 und 3 ml Zulauf 2 vorgelegt und unter Rühren bei 70 °C 10 min anpolymerisiert. Dann werden Zulauf 1 in 4 h und Zulauf 2 in 6 h zugegeben und noch 1 h nachgerührt. Nach Abdestillieren des Isopropanols erhält man eine leicht trübe Lösung eines Polymeren vom K-Wert 22.

Verwendungsbeispiel und Vergleich über das unterschiedliche Korrosionsverhalten:

Nach folgender Rezeptur für kosmetische Haarschäume werden 4 Mischungen hergestellt (jeweils Gewichtsteile):

| | |
|---|---|
| Kationisches Polymer (bezogen auf Feststoff) | 1,2 |
| Fettalkoholethoxylat mit 25 EO (Ceteareth 25) | 0,2 |
| Nonylphenolethoxylat mit 14 EO (Nonoxynol 14) | 0,1 |
| Fettsäurediethanolamid (Cocamide-DEA) | 0,1 |
| Parfüm/Nonoxynol 14 1 : 2 | 0,3 |
| Wasser, dest. | 78,1 |
| Ethanol, abs. | 10,0 |
| Propan/Butan (40 : 60) | 10,0 |

Als kationisches Polymer werden eingesetzt

a) ein handelsübliches Copolymer aus Vinylpyrrolidon und 3-Methyl-1-vinyl-imidazoliumchlorid (70 : 30)

b) wie a) im Verhältnis (50 : 50)

c) erfindungsgemäßes Copolymer Beispiel 6

d) ein handelsübliches Copolymerisat aus Vinylpyrrolidon und β-(Trimethylammonium)-ethylmethacrylat-methosulfat (85 : 15).

Die Mischung wird in Weißblechdosen, wie sie in der Kosmetik bei nichtkorrosiven Formulierungen üblich sind, abgefüllt und bei 40 °C gelagert. Nach 4, 8 und 12 Wochen wird die Dose auf Korrosion untersucht.

Die Ergebnisse sind in der folgenden Tabelle zusammengestellt:

Tabelle

| | a) | b) | c) | d) |
|---|---|---|---|---|
| 4 Wochen | an der Bodennaht u. im Gasraum Korrosion | an der Bodennaht u. im Gasraum Korrosion | keine Korrosion | an der Bodennaht, im Gasraum Korrosion |
| 8 Wochen | am Falz, im Gasraum, am Ventilteller Korrosion | durchgerostet | im Gasraum leichte Korrosion | an Bodennaht im Gasraum u. am Ventilteller Korrosion |
| 12 Wochen | am Falz, im Gasraum, am Ventilteller starke Korrosion | durchgerostet | im Gasraum leichte Korrosion | an Bodennaht, im Gasraum u. am Ventilteller Korrosion |

Für die praktische Verwendung ergibt sich im Hinblick auf das Korrosionsverhalten eine deutliche Überlegenheit des erfindungsgemäßen Polymeren.

## Patentansprüche

1. Wasserlösliche kationische Homopolymere, erhalten aus wasserlöslichen Monomeren mit einer polymerisierbaren olefinischen Doppelbindung und einem quartären Stickstoffatom der Formel I

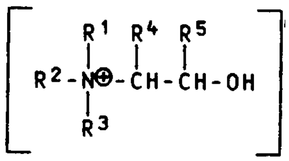 A⊖ I,

in der $R^1$ einen Alkenylrest mit 2 bis 4 C-Atomen oder einen Rest der Formel II

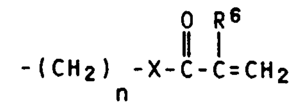 II,

in der für n eine ganze Zahl von 1 bis 18, für X ein Sauerstoffatom oder eine NH-Gruppe und für $R^6$ ein Wasserstoffatom oder Methyl stehen, $R^2$ einen Alkylrest mit 1 bis 18 C-Atomen oder einen Hydroxyalkylrest mit 2 bis 18 C-Atomen,
$R^3$ einen Alkenylrest mit 2 bis 4 C-Atomen oder einen Alkylrest mit 1 bis 18 C-Atomen oder einen Hydroxyalkylrest mit 2 bs 18 C-Atomen bedeuten oder die Reste $R^1$, $R^2$ und $R^3$ zusammen mit dem quartären Stickstoffatom einen 5- oder 6-gliedrigen heterocylischen Ring bilden, der durch eine polymerisierbare olefinische Doppelbindung substuiert ist und ggf. ein weiteres Stickstoffatom und ggf. ein oder zwei Alkylreste mit 1 bis 4 C-Atomen als Substituenten enthält, und $R^4$ und $R^5$ ein Wasserstoffatom oder einen Alkylrest mit 1 bis 28 C-Atomen oder eine durch einen Alkoxyrest mit 1 bis 10 C-Atomen im Alkyl substituierte Methylgruppe bedeuten
und A⊖ für das Äquivalent eines Anions außer Halogenid steht, durch radikalische Polymerisation.

**2.** Wasserlösliche Homopolymere, erhalten aus Monomeren der Formel I nach Anspruch 1, in der $R^1$ Allyl oder Methallyl oder einen Rest der Formel II mit den in Anspruch 1 genannten Bedeutungen, wobei für n eine Zahl von 2 bis 6 steht, $R^2$ einen Alkylrest mit 1 bis 4 C-Atomen oder einen Hydroxyalkylrest mit 2 bis 4 C-Atomen, $R^3$ Allyl oder Methallyl oder einen Alkylrest mit 1 bis 4 C-Atomen oder einen Hydroxyalkylrest mit 2 bis 4 C-Atomen bedeuten oder $R^1$, $R^2$ und $R^3$ zusammen mit dem quartären Stickstoffatom 2- oder 4-Vinylpyridin oder N-Vinylimidazol bilden, ggf. durch einen Methylrest substituiert, und für $R^4$ und $R^5$ ein Wasserstoffatom, ein Alkylrest mit 1 bis 16 C-Atomen oder eine durch einen Alkoxyrest mit 1 bis 10 C-Atomen im Alkyl substituierte Methylgruppe stehen, wobei höchstens einer der Reste $R^4$ oder $R^5$ durch einen organischen Rest ersetzt sein kann, und A⊖ für das Äquivalent eines Anions außer Halogenid steht, durch radikalische Polymerisation.

**3.** Wasserlösliche Copolymerisate, erhalten aus 2 bis 98 Gew.% eines Monomeren der Formel I, wie in Anspruch 1 oder 2 definiert, und 98 bis 2 Gew.% eines polymerisierbaren wasserlöslichen ungesättigten Comonomeren, bezogen auf das Gesamtgewicht der Monomeren, durch radikalische Polymerisation.

**4.** Wasserlösliche Copolymerisate nach Anspruch 3, dadurch gekennzeichnet, daß als wasserlösliches Comonomer ein Hydroxyalkylacrylat oder Hydroxyalkylmethacrylat mit 2 bis 4 C-Atomen im Hydroxyalkylrest oder ein N-Vinylamid, ausgewählt aus der Gruppe N-Vinylpyrrolidon, N-Vinylcaprolactam, N-Vinylformamid, N-Vinylacetamid und N-Methyl-N-vinyl-acetamid copolymerisiert wird.

**5.** Verfahren zur Herstellung von Homopolymeren und Copolymeren nach den Ansprüchen 1 bis 4, dadurch gekennzeichnet, daß die Polymerisation in wäßriger Lösung in Gegenwart von radikalbildenden Initiatoren in einer Menge von 0,1 bis 3 %, bezogen auf das Gewicht der Monomeren, und bei Temperaturen von 40 bis 100 °C durchgeführt wird.

**6.** Verwendung der Polymeren nach Anspruch 1 bis 5 als Leitfähigkeitsharze.

**7.** Verwendung der Polymeren nach Anspruch 1 bis 5 als Flockungsmittel.

**8.** Verwendung der Polymeren nach Anspruch 1 bis 5 in kosmetischen Zubereitungen.

## Claims

1. A water-soluble cationic homopolymer obtained from a water-soluble monomer having a polymerizable olefinic double bond and a quaternary nitrogen atom, of the formula I

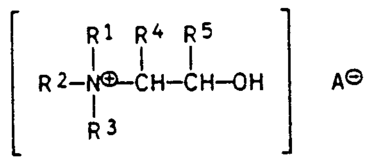

I

where $R^1$ is alkenyl of 2 to 4 carbon atoms or a radical of the formula II

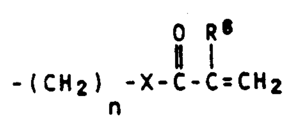

II

where n is an integer from 1 to 18, X is oxygen or an NH group and $R^6$ is hydrogen or methyl, $R^2$ is alkyl of 1 to 18 carbon atoms or hydroxyalkyl of 2 to 18 carbon atoms, $R^3$ is alkenyl of 2 to 4 carbon atoms or alkyl of 1 to 18 carbon atoms or hydroxyalkyl of 2 to 18 carbon atoms, or the radicals $R^1$, $R^2$ and $R^3$ together with the quaternary nitrogen atom form a 5-membered or 6-membered heterocyclic ring which is substituted by a polymerizable olefinic double bond and may contain a further nitrogen atom and may contain one or two alkyl radicals of 1 to 4 carbon atoms as substituents, and $R^4$ and $R^5$ are each hydrogen or alkyl of 1 to 28 carbon atoms or are each methyl which is substituted by alkoxy where alkyl is of 1 to 10 carbon atoms, and $A^\ominus$ is one equivalent of an anion, except for halide, by free radical polymerization.

2. A water-soluble homopolymer obtained from a monomer of the formula I as claimed in claim 1, where $R^1$ is allyl or methallyl or a radical of the formula II having the meanings stated in claim 1, and n is from 2 to 6, $R^2$ is alkyl of 1 to 4 carbon atoms or hydroxyalkyl of 2 to 4 carbon atoms, $R^3$ is allyl or methallyl or alkyl of 1 to 4 carbon atoms or hydroxyalkyl of 2 to 4 carbon atoms, or $R^1$, $R^2$ and $R^3$ together with the quaternary nitrogen atom form 2- or 4-vinylpyridine or N-vinylimidazole which may be substituted by methyl, and $R^4$ and $R^5$ are each hydrogen, alkyl of 1 to 16 carbon atoms or methyl which is substituted by alkoxy where alkyl is of 1 to 10 carbon atoms, and not more than one of the radicals $R^4$ and $R^5$ may be replaced by an organic radical, and $A^\ominus$ is one equivalent of an anion except for halide, by free radical polymerization.

3. A water-soluble copolymer obtained from 2-98% by weight of a monomer of the formula I as defined in claim 1 or 2 and 98-2% by weight of a polymerizable water-soluble unsaturated comonomer, the percentages being based on the total weight of the monomers, by free radical polymerization.

4. A water-soluble copolymer as claimed in claim 3, wherein a hydroxyalkyl acrylate or hydroxyalkyl methacrylate where hydroxyalkyl is of 2 to 4 carbon atoms or an N-vinylamide selected from the group consisting of N-vinylpyrrolidone, N-vinylcaprolactam, N-vinylformamide, N-vinylacetamide and N-methyl-N-vinylacetamide is copolymerized as the water-soluble comonomer.

5. A process for the preparation of a homopolymer or copolymer as claimed in claim 1 or 2 or 3 or 4, wherein the polymerization is carried out in aqueous solution in the presence of a free radical initiator in an amount of from 0.1 to 3%, based on the weight of the monomers, and at from 40 to 100 °C.

6. Use of a polymer as claimed in claim 1 or 2 or 3 or 4 or 5 as a conductivity resin.

7. Use of a polymer as claimed in claim 1 or 2 or 3 or 4 or 5 as a flocculant.

8. Use of a polymer as claimed in claim 1 or 2 or 3 or 4 or 5 in a cosmetic formulation.

## Revendications

**1.** Homopolymères cationiques hydrosolubles, obtenus par polymérisation radicalaire à partir de monomères hydrosolubles renfermant une double liaison oléfinique polymérisable et un atome d'azote quaternaire, de formule I

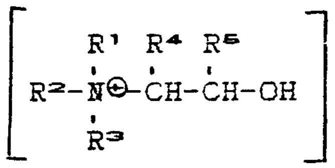

$A^{\ominus}$ I

dans laquelle $R^1$ represente un reste alkylène à 2-4 atomes de carbone ou un reste de formule II

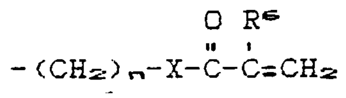

II

dans laquelle n est mis pour un nombre entier de 1 à 18, X pour un atome d'oxygène ou un groupement NH et $R^6$ pour un atome d'hydrogène ou un radical méthyle,
$R^2$ représente un reste alkyle à 1-18 atomes de carbone ou un reste hydroxyalkyle à 2-18 atomes de carbone,
$R^3$ représente un reste alcényle à 2-4 atomes de carbone, un reste alkyle à 1-18 atomes de carbone ou un reste hydroxyalkyle à 2-18 atomes de carbone, ou les restes $R^1$, $R^2$ et $R^3$ forment ensemble, avec l'atome d'azote quaternaire, un noyau penta- ou hexagonal hétérocyclique qui est substitué par une double liaison oléfinique polymérisable et contient éventuellement un autre atome d'azote et éventuellement un ou deux restes alkyle à 1-4 atomes de carbone en tant que substituants,
et $R^4$ et $R^5$ représentent chacun un atome d'hydrogène, un reste alkyle à 1-10 atomes de carbone ou un groupement méthyle substitué par un reste alcoxy à 1-10 atomes de carbone dans le radical alkyle, et $A^{\ominus}$ est mis pour l'équivalent d'un anion en dehors d'un halogénure.

**2.** Homopolymères hydrosolubles, obtenus par polymérisation radicalaire à partir de monomères de formule I selon la revendication 1, dans laquelle $R^1$ est un reste allyle ou méthallyle ou un reste de formule II avec les significations spécifiées dans la revendication I, n étant mis pour un nombre de 2 à 6, $R^2$ représentant un reste alkyle à 1-4 atomes de carbone ou un reste hydroxyalkyle à 2-4 atomes de carbone, $R^3$ représentant un reste allyle ou méthallyle, un reste alkyle à 1-4 atomes de carbone ou un reste hydroxyalkyle à 2-4 atomes de carbone, ou $R^1$, $R^2$ et $R^3$ formant ensemble, avec l'atome d'azote quaternaire, un reste 2- ou 4-vinylpyridine ou N-vinylimidazole éventuellement substitué par un reste méthyle, et $R^4$ et $R^5$ étant mis chacun pour un atome d'hydrogène, un reste alkyle à 1-16 atomes de carbone ou un groupement méthyle substitué par un reste alcoxy à 1-10 atomes de carbone dans le radical alkyle, l'un au maximum des restes $R^4$ ou $R^5$ pouvant être remplacé par un reste organique, et $A^{\ominus}$ étant mis pour l'équivalent d'un anion en dehors d'un halogénure.

**3.** Copolymères hydrosolubles, obtenus par polymérisation radicalaire à partir de 2 à 98% en poids d'un monomère de formule I, tel que défini dans la revendication 1 ou 2, et de 98 à 2% en poids d'un comonomère insaturé hydrosoluble polymérisable, par rapport au poids total des monomères.

**4.** Copolymères hydrosolubles selon la revendication 3, caractérisés en ce qu'on copolymérise, en tant que comonomère hydrosoluble, un acrylate d'hydroxyalkyle ou un méthacrylate d'hydroxyalkyle à 2-4 atomes de carbone dans le reste hydroxyalkyle, ou un N-vinylamide choisi dans le groupe de la N-vinylpyrrolidone, du N-vinylcaprolactame, du N-vinylformamide, du N-vinylacétamide et du N-méthyl-N-vinylacétamide.

**5.** Procédé de préparation d'homopolymères et de copolymères selon l'une quelconque des revendications 1 à 4, caractérisé en ce que la polymérisation est conduite en solution aqueuse, en présence d'initiateurs radicalaires dans une proportion de 0,1 à 3% par rapport au poids des monomères, et à des températures de 40 à 100 °C.

6. Utilisation des polymères selon l'une quelconque des revendications 1 à 5 comme résines de conductivité.

7. Utilisation des polymères selon l'une quelconque des revendications 1 à 5 comme floculants.

8. Utilisation des polymères selon l'une quelconque des revendications 1 à 5 dans des préparations cosmétiques.